# EUROPÄISCHE PATENTSCHRIFT

(11) **EP 1 858 490 B1**
(45) Veröffentlichungstag und Bekanntmachung des Hinweises auf die Patenterteilung: **11.04.2012**
(21) Anmeldenummer: 06706994.8
(22) Anmeldetag: 16.02.2006
(51) Int. Cl.: A61K 9/22, A61K 31/53

(54) **ARZNEIFORMEN MIT VERBESSERTEN PHARMAKOKINETISCHEN EIGENSCHAFTEN**
PHARMACEUTICAL FORMS WITH IMPROVED PHARMACOKINETIC PROPERTIES
FORMES PHARMACEUTIQUES A PROPRIETES PHARMACOCINETIQUES AMELIOREES

(30) Priorität: 01.03.2005 DE 102005009240
(43) Veröffentlichungstag der Anmeldung: 28.11.2007
(60) Teilanmeldung: 11172872.1
(73) Patentinhaber: Bayer Pharma Aktiengesellschaft, 13353 Berlin (DE)
(72) Erfinder: SERNO, Peter, 51467 Bergisch Gladbach (DE); HEINIG, Roland, 42115 Wuppertal (DE); PAULI, Kerstin, 44139 Dortmund (DE); HAYAUCHI, Yutaka, 51373 Leverkusen (DE)
(86) Internationale Anmeldenummer: PCT/EP2006/001393
(87) Internationale Veröffentlichungsnummer: WO 2006/092207

(56) Entgegenhaltungen:
- WO-A-2005/110419
- DE-A1- 10 232 113
- DOBETTI L: "FAST-MELTING TABLETS: DEVELOPMENTS AN TECHNOLOGIES" PHARMACEUTICAL TECHNOLOGY INTERNATIONAL, INGLEWOOD, CA, US, Bd. 12, Nr. 9, 2000, Seiten 32,34-36,38,40,4, XP001145817 ISSN: 0164-6826
- "Quick-Dissolving Tablets Made Easy with Pharmaburst" SPECIAL DELIVERY; SPI PHARMA, [Online] 2002, Seiten 1-4, XP002382234 Gefunden im Internet: URL:http://www.spipharma.com/images/SDIssu e3.pdf> [gefunden am 2006-05-11]
- YOUNG J M: "Vardenafil" EXPERT OPINION ON INVESTIGATIONAL DRUGS 01 OCT 2002 UNITED KINGDOM, Bd. 11, Nr. 10, 1. Oktober 2002 (2002-10-01), Seiten 1487-1496, XP002382235 ISSN: 1354-3784

## Beschreibung

Die vorliegende Anmeldung betrifft neue Arzneimittelformulierungen von Vardenafil die sich schnell im Mund auflösen und die zu einer Erhöhung der Bioverfügbarkeit und zu einem plateau-artigen Verlauf der Plasmakonzentration führen, sowie Verfahren zu deren Herstellung.

Imidazotriazinon-Derivate, wie Vardenafil sowie dessen Verwendung als cGMP Phosphodiesterase-Inhibitor und dessen Wirkungsspektrum sind bekannt (z.B. WO 99/24433) und unter dem Namen Levitra® im Markt erhältlich. Der therapeutische Einsatz von Vardenafil wird jedoch durch dessen geringe Bioverfügbarkeit von etwa 14 % sowie den raschen Abfall der Plasmakonzentration etwa 1 Stunde nach der Applikation von Vardenafil beeinträchtigt. Eine geringe Bioverfügbarkeit hat grundsätzlich hohe Variabilität der Plasmakonzentrationen zwischen verschiedenen Individuen zur Folge, ferner muss die Dosierung erhöht werden, um eine bestimmte Exposition zu erreichen. Das rasche Abfallen der Plasmakonzentration etwa 1 Stunde nach der oralen Einnahme von Vardenafil birgt das Risiko, dass nachfolgend eine geringere therapeutische Wirksamkeit resultiert. Die Patienten müssen daher den Zeitpunkt der Einnahme genau planen, um in den Vorteil hoher Plasmakonzentrationen zu gelangen.

Aus diesen Gründen wurde versucht, eine Arzneiform von Vardenafil zu finden, welche eines der genannten Probleme löst. Die Anmeldung US 2003/0134861 Al beschreibt Formulierungen zur transmucosalen Applikation von Phosphodiesterase - Inhibitoren, beispielsweise bukkale Arzneiformen oder sublinguale Tabletten. Wie die Vergleichsbeispiele 1 bis 3 jedoch zeigen, resultieren im Fall von Vardenafil nach Applikation über die orale Schleimhaut sehr unbefriedigende Plasmakonzentrationen mit hoch variabler, unvollständiger und langsamer Absorption des Wirkstoffes.

Daneben wurden Arzneiformen mit verzögerter Freisetzung von cGMP Phosphodiesterase - Inhibitoren beschrieben (WO 00/24383). Derartige Arzneiformen können das Problem des raschen Abfallens der Plasmakonzentrationen lösen. Retardarzneiformen sind jedoch groß und sind für einen Teil der Patienten schwer zu schlucken. Ferner beheben sie in keiner Weise das Problem der geringen Bioverfügbarkeit von Vardenafil.

Ferner wurden im Mund zerfallende Arzneiformen von cGMP Phopshodiesterase - Inhibitoren beschrieben. US 6,221,402 beschreibt eine Arzneiform unter anderem für Antiimpotenz - Wirkstoffe, in der der wirkstoffhaltige Kern unter anderem mit einem Speichel - unlöslichen Polymer überzogen ist. US 2002/0002172 beschreibt eine im Mund zerfallende Arzneiform der cGMP Phosphodiesterase - Inhibitors Sildenafil, die den Wirkstoff als freie Base geringer Wasserlöslichkeit enthält. Derartige im Mund zerfallende Arzneiformen haben den Vorteil der leichten Einnehmbarkeit durch den Patienten, da die Arzneiform bereits im Mund zerfällt. Sie bewirken jedoch weder eine Erhöhung der Bioverfügbarkeit noch ein längeres Anhalten von Plasmakonzentrationen. Da die zerfallene Arzneiform vom Patienten innerhalb kurzer Zeit heruntergeschluckt wird, erfolgt eine Auflösung des Wirkstoffes wie bei einer konventionellen Schlucktablette erst im Magen. Es resultiert daher im besten Fall eine ähnliche Bioverfügbarkeit wie nach Applikation konventioneller Schlucktabletten.

Überraschenderweise wurden nun im Mund zerfallende Arzneiformen von Vardenafil gefunden, die zu einer Erhöhung der Bioverfügbarkeit und zu einem plateau-artigen Verlauf der Plasmakonzentration führen. Die erfindungsgemäßen Formulierungen besitzen im Vergleich zu einer üblichen, mit Wasser zu schluckenden Tablette eine deutlich erhöhte Bioverfügbarkeit. Dabei werden die höheren Plasmakonzentrationen insbesondere in dem Zeitraum erreicht, in dem bei der üblichen Schlucktablette bereits wieder ein Abfallen der Plasmakonzentrationen eintritt, also beispielsweise im Bereich 0 bis 5 Stunden nach Erreichen der maximalen Plasmakonzentration. Als Folge ist bei gleicher Dosis mit einer verbesserten Wirksamkeit in dieser Zeit zu rechnen. Insbesondere die Erhöhung der Plasmakonzentrationen noch mehrere Stunden nach Applikation einer besonders schnell zerfallenden und freisetzenden Arzneiform stellt eine unerwartete Entdeckung dar, da man durch Beschleunigen der Wirkstoffauflösung eher ein rascheres Anfluten und schnelleres Abfallen der Vardenafil - Plasmakonzentrationen erwartet hätte.

Gegenstand der Erfindung ist daher eine Vardenafil enthaltende Arzneimittel-Formulierung die dadurch gekennzeichnet ist, dass die Löslichkeit der eingesetzten Form von Vardenafil in einer kleinen Menge wässriger Flüssigkeit ausreichend hoch ist und die Lösegeschwindigkeit aus der im Mund zerfallenden Formulierung ausreichend schnell ist. Es wurde gefunden, dass dies gewährleistet ist, wenn die Arzneimittelformulierung a) Vardenafil Hydrochlorid Trihydrat und b) Zuckeralkohol enthält und sich mindestens 80 % der Vardenafil - Dosis aus der eingesetzten Substanzform bei 25°C in 10 ml physiologischer Kochsalzlösung auflöst und wenn die Freisetzungsrate aus der Arzneiform in 900 ml physiologischer Kochsalzlösung innerhalb der ersten 5 Minuten mindestens 70 % beträgt (37°C, USP Blattrührerapparatur, 50 Umdrehungen pro Minute).

Ein weiterer Aspekt der Erfindung ist die Anwendung eines optimalen Einnahmeverfahrens für die erfindungsgemäßen Zubereitungen. Üblicherweise werden transmukosale Arzneiformen möglichst lange und intensiv mit der Schleimhaut in Kontakt gebracht, beispielsweise durch Ankleben eines wirkstoffhaltigen Filmes auf die Mundschleimhaut. Wenn dies nicht erwünscht oder möglich ist, werden Tabletten im Allgemeinen mit etwas Flüssigkeit geschluckt. Es wurde gefunden, dass beide Verfahrensweisen die erzielbare Bioverfügbarkeit von Vardenafil beeinträchtigen. Dagegen kann eine Erhöhung der Bioverfügbarkeit von Vardenafil erreicht werden, wenn der Patient die erfindungsgemäße Arzneiform in die Mundhöhle einlegt, deren Zerfall im Mund abwartet und nachfolgend die entstehende Lösung oder Suspension schluckt. Die erfindungsgemäßen Arzneiformen werden daher in einem Primärpackmittel, beispielsweise einer Kunststoffflasche oder einer Blisterpackung, verpackt, und mit einem Etikett oder Beipackzettel versehen, in dem die genannte Einnahmeweise beschrieben ist.

Im Einzelnen ist zur Herstellung der erfindungsgemäßen Formulierungen Vardenafil in Form von Vardenafil Hydrochlorid Trihydrat enthalten.. Zur Erreichung der erfindungsgemäßen Lösungsgeschwindigkeit ist es ferner vorteilhaft, wenn das Vardenafil - Salz in der Arzneiform in gemahlener, amorpher oder bereits gelöster Form enthalten ist. Bevorzugt wird das Vardenafil-Salz in mikronisierter Form zugesetzt, deren mittlere Teilchengröße kleiner als 20 µm ist. Der Gehalt von Vardenafil - Salz in der schnell im Mund zerfallenden Arzneiform liegt bevorzugt zwischen 0,8 % und 25 % (berechnet als Vardenafil Base).

Das Vardenafil - Salz wird in einem der bekannten Verfahren in eine schnell im Mund zerfallende Arzneiform überführt. Unter schnell im Mund zerfallender Arzneiform wird hierbei verstanden, dass die Zerfallszeit der Arzneiform (Methode der Europäischen Pharmakopoe) kürzer als 3 Minuten, vorzugsweise kürzer als 1 Minute ist. Hierzu eignen sich das Mischen des Wirkstoffes mit Zuckern, Zuckeralkoholen, Sprengmitteln oder anderen Zerfallsförderern sowie weiteren Hilfsstoffen wie Tenside, Schmiermittel, Fließregulierungsmittel Geschniackstoffe, Farbstoffe oder Füllstoffe und Verpressen auf einer Tablettenmaschine. Die erfindungsgemäßen Formulierungen enthalten Zuckeralkoholen wie Mannit oder Sorbit, insbesondere in einer Konzentration (bezogen auf die fertige Tablette) von 40 % bis 99 %. Das Vardenafil - Salz kann zusammen mit Hilfsstoffen wie Zuckern, Zuckeralkoholen, Polymeren oder Tensiden in einem wässrigen Lösungsmittel gelöst oder suspendiert werden, die Lösung oder Suspension wird in Blisternäpfe dosiert und einem Gefriertrocknungsprozess unterworden. Alternativ kann das Vardenafil - Salz zusammen mit Hilfsstoffen wie Filmbildnern, Weichmachern, Geschmacks- und Farbstoffen in einem organischen Lösungsmittel gelöst oder suspendiert werden und zu einem Film verarbeitet werden. Auch eine lösungsmittelfreie Filmherstellung mit schmelzbaren Filmformulicrungen ist möglich. Nach der Herstellung werden die Filme auf die einer Einzeldosis entsprechenden Stücke geschnitten.

### Vergleichsbeispiel 1

### Geringe und langsame Absorption von Vardenafilhydrochlorid aus einer Arzneiform zur Applikation über die Mundschleimhaut

30 mg Vardenafilhydrochlorid, 54 mg Methylparahydroxybenzoat, 6 mg Propylparahydroxybenzoat und 9 g Saccharose werden in etwa 20 g Wasser gelöst. Der pH wird mit 20 %iger Milchsäurelösung auf 3.9 eingestellt. Danach wird mit Wasser auf die Gesamteinsatzmenge von 33,405 g aufgefüllt. 10 Probanden werden jeweils 11.97 g dieser Lösung (entsprechend 10 mg Vardenafil) für 15 Minuten sublingual appliziert. Als Vergleich wird im Cross-over - Verfahren eine übliche, mit Wasser zu schluckende Tablette gegeben, die aus folgenden Bestandteilen besteht: 11,852 mg Vardenafil Hydrochlorid Trihydrat (entsprechend 10 mg Vardenafil), 105,023 mg Mikrokristalline Cellulose, 6,25 mg Quervernetztes Polyvinylpyrrolidon, 0,625 mg kolloidales Siliciumdioxid, 1,25 mg Magnesiunistearat, 2,391 mg Hypromellose, 0,797 mg Macrogol 400, 0,653 mg Titandioxid, 0,133 mg gelbes Eisenoxid und 0,011 mg rotes Eisenoxid. Im Vergleich zu dieser als Referenz dienenden Standardtablette ergibt sich für die sublingual applizierte Lösung lediglich eine relative Bioverfügbarkeit von 24,6 %.

### Vergleichsbeispiel 2

### Geringe und langsame Absorption von Vardenafil aus einer Arzneiform zur Applikation über die Mundschleimhaut

2 g Vardenafil, 0,1g Ascorbylpalmitat, 0,5 g α-Tocopherol und 7,8 g Trometamol werden in 250 g Polysorbat 20, 400 g 1,2 Propylenglykol, 250 g Ethanol 96 %, 35,8 g 1 M Salzsäure und 52.6 g Wasser gelöst. 5 ml dieser Lösung (entsprechend 10 mg Vardenafil) werden 10 Probanden 15 Minuten lang sublingual gegeben. Im Cross-over Vergleich erhalten die Probanden die in Vergleichsbeispiel 1 beschriebene 10 mg Vardenafil Tablette als Referenz, die mit Wasser geschluckt wird. Die relative Bioverfügbarkeit der sublingual applizierten Lösung beträgt 18,9 %.

### Vergleichsbeispiel 3

### Geringe und langsame Absorption von Vardenafilmesilat aus einer Arzneiform zur Applikation über die Mundschleimhaut

10 Probanden erhalten jeweils für 15 Minuten sublingual eine Tablette appliziert, die aus 2,39 mg Vardenafilmonomesilat, 0,0986 mg Methansulfonsäure, 20 mg Mannit, 2 mg Croscarmellose Natrium, 25,3 mg Mikrokristalliner Cellulose, 1 mg Magnesiumstearat und 0,25 mg hochdispersem Siliziumdioxid besteht. Die Tablette hat eine Zerfallszeit von 4 Minuten. Im Cross-over Vergleich erhalten die Probanden die in Vergleichsbeispiel 1 beschriebene 10 mg Vardenafil Tablette als Referenz, die mit Wasser geschluckt wird. Die dosisnormierte relative Verfügbarkeit der Sublingualtablette beträgt 43,9 %.

### Vergleichsbeispiel 4

### Fehlen erhöhter Bioverfügbarkeit bei einer nicht erfindungsgemäßen, schnell im Mund zerfallenden Tablette

11 Probanden erhalten jeweils eine schnell im Mund zerfallende Tablette bestehend aus 10.7 mg Vardenafildihydrat (entsprechend 10 mg Vardenafil), 0,484 mg gelbem Eisenoxid, 0,066 mg rotem Eisenoxid, 1,1 mg Aprikosenaroma, 4,4 mg Aspartam, 6,6 mg Magnesiumstearat und 196,65 mg Pharmaburst^{®} (handelsübliche Hilfsstoffmischung der Firma SPI). Diese schnell im Mund zerfallende Tablette ist nicht erfindungsgemäß, da sich in 10 ml Physiologischer Kochsalzlösung bei 25°C nur etwa 0,1 mg Vardenafildihydrat (entsprechend etwa 1 % der applizierten Dosis) lösen und somit das Löslichkeitskriterium der eingesetzten Wirkstoffform nicht erfüllt wird. Im Cross-over - Vergleich zu der im Vergleichsbeispiel 1 aufgeführten Referenztablette beträgt die relative Bioverfügbarkeit 97,3 %.

### Vergeichsbeispiel 5

### Fehlen erhöhter Bioverfügbarkeit bei einer nicht erfindungsgemäßen, schnell im Mund zerfallenden Tablette

11 Probanden erhalten jeweils eine im Mund zerfallende Tablette bestehend aus 10.7 mg Vardenafildihydrat (entsprechend 10 mg Vardenafil), 5 mg gemahlener Bernsteinsäure, 0,484 mg gelbem Eisenoxid, 0,066 mg rotem Eisenoxid, 1,1 mg Aprikosenaroma, 4,4 mg Aspartam, 6,6 mg Magnesiumstearat und 191,65 mg Pharmaburst^{®} (handelsübliche Hilfsstoffmischung der Firma SPI). Diese schnell im Mund zerfallende Tablette ist nicht erfindungsgemäß, da die Wirkstofffreisetzung in 900 ml physiologischer Kochsalzlösung bei 37°C und 50 Umdrehungen pro Minute in der USP Blattrührerapparatur nur 40 % in 5 Minuten beträgt und somit das erfindungsgemäße Lösungsgeschwindigkeitskriterium nicht erfüllt wird. Im Cross-over - Vergleich zu der im Vergleichsbeispiel 1 aufgeführten Referenztablette beträgt die relative Bioverfügbarkeit 101,8 %.

### Beispiel 6

### Nachweis erhöhter Bioverfügbarkeit bei einer erfindungsgemäßen, schnell im Mund zerfallenden Tablette

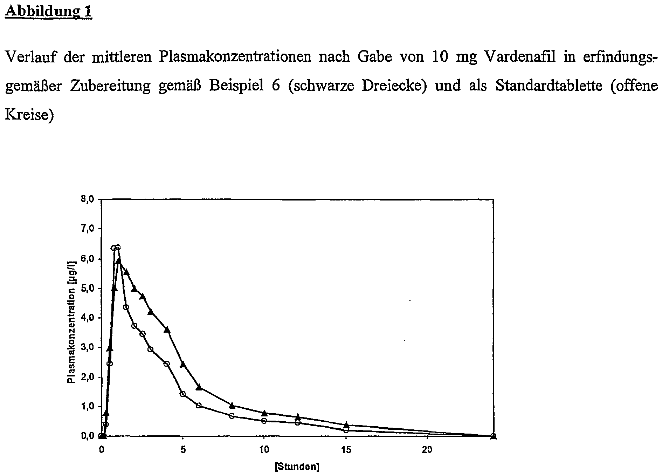

12 Probanden erhalten jeweils eine schnell im Mund zerfallende Tablette bestehend aus 11,85 mg Vardenafil hydrochlorid trihydrat, 0,55 mg gelbem Eisenoxid, 0,075 mg rotem Eisenoxid, 0,75 mg Aprikosenaroma, 0,125 mg Neohesperidin-Dihydrochalcone, 2,50 mg Aspartam 0,625 mg hochdispersem Siliciumdioxid, 3,125 mg Magnesiumstearat und 105,4 mg Pharmaburst^{®}. Vom eingesetzten Wirkstoff lösen sich bei 25°C in 10 ml physiologischer Kochsalzlösung etwa 10,4 mg (entsprechend 8,8 mg Vardenafil) und somit 88 % der Dosis. Die Wirkstofffreisetzung in 900 ml physiologischer Kochsalzlösung bei 37°C und 50 Umdrehungen pro Minute in der USP Blattrührerapparatur beträgt 73 % in 5 Minuten. Somit sind das erfindungsgemäße Löslichkeits- und Lösegeschwindigkeitskriterium erfüllt. Im Vergleich zu der im Vergleichsbeispiel 1 beschriebenen Referenztablette beträgt die relative Bioverfügbarkeit 141 %. Die entsprechenden pharmakokinetischen Parameter sind in Tabelle 1 (Anlage), der Verlauf der mittleren Plasmakonzentrationen in Abbildung 1 (Anlage) vergleichend dargestellt.

### Beispiel 7

### Nachweis erhöhter Bioverfügbarkeit bei einer erfindungsgemäßen, schnell im Mund zerfallenden Tablette

11 Probanden erhalten jeweils eine im Mund zerfallende Tablette bestehend aus 5,93 mg Vardenafil hydrochlorid trihydrat, 0,352 mg gelbem Eisenoxid, 0,048 mg rotem Eisenoxid, 0,48 mg Aprikosenaroma, 0,08 mg Neohesperidin-Dihydrochalcone, 1,60 mg Aspartam 0,40 mg hochdispersem Siliciumdioxid, 2 mg Magnesiumstearat und 69,11 mg Pharmaburst^{®}. Der eingesetzte Wirkstoff löst sich bei 25°C in 10 ml physiologischer Kochsalzlösung zu 91 %. Die Wirkstofffreisetzung in 900 ml physiologischer Kochsalzlösung bei 37°C und 50 Umdrehungen pro Minute in der USP Blattrührerapparatur beträgt 78 % in 5 Minuten. Somit sind das erfindungsgemäße Löslichkeits- und Lösegeschwindigkeitskriterium erfüllt. Als Vergleich wird im Cross-over - Verfahren eine übliche, mit Wasser zu schluckende Tablette gegeben, die aus folgenden Bestandteilen besteht: 5,926 mg Vardenafil Hydrochlorid Trihydrat (entsprechend 5 mg Vardenafil), 75,419 mg Mikrokristalline Cellulose, 4,35 mg Quervernetztes Polyvinylpyrrolidon, 0,435 mg kolloidales Siliciumdioxid, 0,87 mg Magnesiumstearat, 1,664 mg Hypromellose, 0,555 mg Macrogol 400, 0,455 mg Titandioxid, 0,092 mg gelbes Eisenoxid und 0,007 mg rotes Eisenoxid. Im Vergleich zu dieser Referenztablette beträgt die relative Bioverfügbarkeit 149.6%. Noch bis zu 12 Stunden nach Applikation der erfindungsgemäßen Tablette sind die Plasmakonzentrationen höher als die nach Gabe der Standardtablette.

### Beispiel 8

### Nachweis erhöhter Bioverfügbarkeit bei einer erfindungsgemäßen, schnell im Mund zerfallenden Tablette

In einem Pflugscharmischer werden folgende Bestandteile gemischt: 697 g mikronisiertes Vardenafil hydrochlorid Trihydrat, 500 g einer Farbstoff - Vormischung bestehend aus 4,4 % gelbem Eisenoxid, 0,6 % rotem Eisenoxid und 95 % Pharmaburst^{®}, 30 g Aprikosenaroma, 5 g Neohesperidin- Dihydrochalcone, 100 g Aspartam und 3518 g Pharmaburst®. Die Pulvermischung wird in einem Freifallmischer mit 25 g hochdispersem Siliciumdioxid gemischt und durch ein 0,5 mm Sieb gesiebt. Diese Mischung wird mit 125 g Magnesiumstearat in einem Freifallmischer 5 Minuten lang gemischt. Die fertige Pulvermischung wird auf einer Tablettenpresse zu runden Tabletten mit einer Masse von 170 mg, einem Durchmesser von 8 mm und einer Bruchfestigkeit von etwa 35 N verpresst. Als Vergleich wird im Cross-over - Verfahren eine übliche, mit Wasser zu schluckende Tablette gegeben, die aus folgenden Bestandteilen besteht: 23,705 mg Vardenafil Hydrochlorid Trihydrat (entsprechend 20 mg Vardenafil), 141,797 mg Mikrokristalline Cellulose, 8,85 mg Quervernetztes Polyvinylpyrrolidon, 0,885 mg kolloidales Siliciumdioxid, 1,77 mg Magnesiumstearat, lackiert mit: 3,385 mg Hypromellose, 1,128 mg Macrogol 400, 0,925 mg Titandioxid, 0,188 mg gelbes Eisenoxid und 0,015 mg rotes Eisenoxid. Im Vergleich zu dieser Referenztablette beträgt die relative Bioverfügbarkeit 128.2 %.

### Beispiel 9 (Dieses Beispiel fällt nicht in den Umfang der Ansprüche und bildet keinen Teil der Erfindung)

Es werden gemischt und nachfolgend trocken auf einer Walze granuliert: 18,96 kg Vardenafil Hydrochlorid Trihydrat, 76,54 kg Mikrokristalline Cellulose, 20 kg Crospovidone, und 80 kg Calciumsilikat. Das Granulat wird nachgemischt mit: 1 kg hochdisperses Siliciumdioxid, 0,5 kg Sucralose, 1 kg pulverförmiges Orangenaroma und 2 kg gesiebtes Magnesiumstearat. Die fertige Mischung wird auf einer Rundlaufpresse zu Tabletten mit einem Durchmesser von 7 mm und 125 mg Masse verpresst.

### Beispiel 10 (Dieses Beispiel fällt nicht on den Umfang der Ansprüche und bildet keinen Teil der Erfindung)

Die folgenden Bestandteile werden gemischt. 21,4 kg Vardenafil Dihydrat, 60 kg gemahlene Bernsteinsäure, 1,1 kg Sucralose und 342,1 kg Pharmaburst^{®} B2, 13,2 kg gesiebtes Magnesiumstearat und 2,2 kg pulverförmiges Orangenaroma. Die Mischung wird zu Tabletten von 9 mm Durchmesser und 220 mg Masse verpresst (entsprechend einer Dosis von 10 mg Vardenafil). 10 mg Vardenafil und 30 mg Bernsteinsäure lösen sich bei 25°C in 10 ml physiologischer Kochsalzlösung vollständig. Die Lösegeschwindigkeit der Tabletten beträgt 90 % in 5 Minuten in der USP Blattrührerapparatur mit 900 ml physiologischer Kochsalzlösung, 37°C und 50 Umdrehungen pro Minute.

**Tabelle 1**

| Pharmakokinetische Parameter von Vardenafil | | | |
|---|---|---|---|
| | | **A** | **B** |
| | | Erfindungsgemäße, schnell im Mund zerfallende Tablette geo.mean geo.%CV (N=12) | Übliche mit Wasser zu schluckende Tablette geo.mean geo.%CV (N=12) |
| AUC | [µg*h/L] | 32.2 (32.0) | 22.8 (38.2) |
| fᵣₑₗ (A: B) | [%] | 140.9 (120.2 -165.2) | |
| Cₘₐₓ | [µg/L] | 7.51 (43.9) | 7.35 (39.5) |
| tₘₐₓ | [h] | 0.875 (0.50-2.50) | 0.75 (0.50-2.00) |
| t_{1/2} | [h] | 4.12 (22.1) | 4.08 (24.0) |

| | | | |
|---|---|---|---|
| tₘₐₓ als median (minimum - maximum) fᵣₑₗ als Punktschätzer (90% Konfidenzintervall) | | | |

## Patentansprüche

1. Arzneimittelformulierung, die schnell im Mund zerfällt, enthaltend a) Vardenafil Hydrochlorid Trihydrat und b) Zuckeralkohol, **dadurch gekennzeichnet, dass** sich mindestens 80 % der Vardenafil-Dosis aus der eingesetzten Substanzform bei 25°C in 10 ml physiologischer Kochsalzlösung auflöst und die Freisetzungsrate aus der Arzneimittelformulierung in 900 ml physiologischer Kochsalzlösung innerhalb der ersten 5 Minuten in der USP Blattrührerapparatur bei 50 Umdrehungen pro Minute bei 37 °C mindestens 70 % beträgt.

2. Arzneimittelformulierung gemäß Anspruch 1, enthaltend Vardenafil. Hydrochlorid Trihydrat in gemahlener, amorpher oder bereits gelöster Form.

3. Arzneimittelformulierung gemäß Anspruch 1, enthaltend Vardenafil Hydrochlorid Trihydrat in mikronisierter Form mit einer mittleren Partikelgröße von kleiner 20 µm.

4. Arzneimittelformulierung gemäß einem oder mehreren der Ansprüche 1 bis 3, enthaltend 40 % bis 99 % Zuckeralkohole.

5. Arzneimittelformulierung gemäß einem oder mehreren der Ansprüche 1 bis 4, enthaltend Mannitol als Zuckeralkohol.

6. Arzneimittelformulierung gemäß einem oder mehreren der Ansprüche 1 bis 5, enthaltend Sorbitol als Zuckeralkohol.

7. Arzneimittelformulierung gemäß einem oder mehreren der Ansprüche 1 bis 5, enthaltend eine Mischung aus Mannitol und Sorbitol als Zuckeralkohol.

8. Arzneimittelformulicrung gemäß einem oder mehreren der Ansprüche 1 bis 7, enthaltend zwischen 0.8% und 25% Vardenafil berechnet als freie Vardenafil Base.

9. Arzneimittelformulierung gemäß einem der Ansprüche 1 bis 5, in einer Arzneipackung mit einem Hinweis auf dem Beipackzettel, Etikett oder Verpackungskarton, dass die Arzneimittelformulierung in die Mundhöhle eingelegt wird und nach deren Zerfall geschluckt wird.

## Claims

1. Drug formulation which disintegrates rapidly in the mouth and comprises a) vardenafil hydrochloride trihydrate and b) sugar alcohol, **characterized in that** at least 80% of the vardenafil dose in the substance form employed dissolves at 25°C in 10 ml of physiological saline and the rate of release from the drug formulation in 900 ml of physiological saline within the first 5 minutes in the USP paddle stirrer apparatus at 50 rotations per minute at 37 °C is at least 70%.

2. Drug formulation according to Claim 1, comprising vardenafil hydrochloride trihydrate in ground, amorphous or already dissolved form.

3. Drug formulation according to Claim 1, comprising vardenafil hydrochloride trihydrate in micronized form with a mean particle size of less than 20 µm.

4. Drug formulation according to one or more of Claims 1 to 3, comprising from 40% to 99% of sugar alcohols.

5. Drug formulation according to one or more of Claims 1 to 4, comprising mannitol as sugar alcohol.

6. Drug formulation according to one or more of Claims 1 to 5, comprising sorbitol as sugar alcohol.

7. Drug formulation according to one or more of Claims 1 to 5, comprising a mixture of mannitol and sorbitol as sugar alcohol.

8. Drug formulation according to one or more of Claims 1 to 7, comprising between 0.8% and 25% of vardenafil, calculated as free vardenafil base.

9. Drug formulation according to any of Claims 1 to 5 in a drug packaging with a note on the information leaflet, label or packaging box that the drug formulation is to be inserted into the oral cavity and, after its disintegration, to be swallowed.

## Revendications

1. Formulation médicamenteuse qui se décompose rapidement en bouche, contenant a) du chlorhydrate de Vardenafil trihydraté et b) un alcool de sucre, **caractérisée en ce qu'**au moins 80% de la dose de Vardenafil de la forme de substance utilisée se dissout à 25°C dans 10 ml de solution saline physiologique et le taux de libération de la formulation médicamenteuse dans 900 ml de solution saline physiologique pendant les 5 premières minutes dans un appareil à agitateur à pale USP à 50 t/min à 37°C est d'au moins 70%.

2. Formulation médicamenteuse selon la revendication 1, contenant du chlorhydrate de Vardenafil trihydraté sous forme broyée, amorphe ou déjà dissoute.

3. Formulation médicamenteuse selon la revendication 1, contenant du chlorhydrate de Vardenafil trihydraté sous forme micronisée, présentant une grosseur moyenne des particules inférieure à 20 µm.

4. Formulation médicamenteuse selon l'une ou plusieurs des revendications 1 à 3, contenant 40% à 99% d'alcools de sucre.

5. Formulation médicamenteuse selon l'une ou plusieurs des revendications 1 à 4, contenant du mannitol comme alcool de sucre.

6. Formulation médicamenteuse selon l'une ou plusieurs des revendications 1 à 5, contenant du sorbitol comme alcool de sucre.

7. Formulation médicamenteuse selon l'une ou plusieurs des revendications 1 à 5, contenant un mélange de mannitol et de sorbitol comme alcool de sucre.

8. Formulation médicamenteuse selon l'une ou plusieurs des revendications 1 à 7, contenant entre 0,8% et 25% de Vardenafil, calculé sous forme de Vardenafil base libre.

9. Formulation médicamenteuse selon l'une quelconque des revendications 1 à 5, dans un emballage médical avec une indication sur la notice, l'étiquette ou le carton d'emballage que la formulation médicamenteuse est introduite dans la cavité buccale et avalée après sa décomposition.
